# EUROPEAN PATENT SPECIFICATION

(11) **EP 1 444 975 B1**
(45) Date of publication and mention of the grant of the patent: **10.08.2011**
(21) Application number: 04001866.5
(22) Date of filing: 29.01.2004
(51) Int. Cl.: A61Q 11/00, A61K 8/73

(54) **Personal care compositions with portable packs**
Körperpflegende Zusammensetzungen mit tragbaren Verpackungen
Compositions de soins personels avec emballages portatifs

(30) Priority: 31.01.2003 NZ 52394603
(43) Date of publication of application: 11.08.2004
(60) Divisional application: 10000193.2
(73) Proprietor: PBL Technology Limited, Auckland 1003 (NZ)
(72) Inventor: Alexander, Carl Ernest, Kent TN26 3JB (GB); Grayson, Francis William, Auckland 1005 (NZ)
(74) Representative: Bentham, Andrew

(56) References cited:
- EP-A- 0 711 544
- EP-A1- 0 548 814
- DE-A- 4 238 421
- DE-U- 20 014 157
- FR-A1- 2 822 700
- GB- - 1 206 163
- GB-A- 720 126
- JP-A- 2002 179 140
- US-A- 5 961 990
- US-A- 6 077 544
- US-A1- 2003 003 059

## Description

### FIELD

This invention relates to dental hygiene compositions and packs/kits for human use; to those compositions and packs when adapted for use along with a brush or the like as an hygiene applicator, and particularly to compositions in integral or single-dose form, distributed in portable packs.

### BACKGROUND

There have not been any significant changes in the presentation of dentifrices, toothpastes, or other compounds usable as tooth cleaning agents for some time. The dental health of children in particular remains under threat, especially with the availability of sweetened drinks and snack foods in the Western world. Recent World Health Organisation figures for the incidence of dental caries within children shows a high incidence of caries in even some developed countries.

Known tooth-cleaning preparations include the currently successful toothpastes. A typical dentifrice formulation includes, in a watery base, a thickener, an abrasive, a flavour such as peppermint, and a detergent which has surface-active and foaming properties. The toothpaste has a white appearance if the abrasive is a mineral powder with a refractive index unlike water. Gel toothpastes employ a cellulose derivative such as carboxymethyl cellulose as a thickener or viscosity raising material, and hydrated silica (having a refractive index like that of water (1.33)) as a dentifrice or abrasive, so that they can be extruded on to a brush as a clear or artificially coloured viscous material and stay on the brush until a lather is generated.

Common toothpastes usually contain gelling agents or water-soluble polymers, serving the purpose of thickeners and binders with several purposes. Settling of the contents during storage within the tube is undesirable. The toothpaste must remain able to be extruded from the tube, yet be tacky enough to lie upon the brush before use, or the teeth during use, but not be so tacky as to leave a trail.

Preferred thickeners include carboxymethyl cellulose and other cellulose ethers. Carrageenan, xanthan and polyacrylates are used much less widely. The subset of toothpastes sold as gels are clear in appearance and are not sufficiently solid to form separate beads capable of remaining apart after storage for periods of typically one year or more.

Manufacture of ordinary toothpaste then filling it into deformable toothpaste tubes is a complex process. Ordinary toothpaste in the tube is not adapted for carriage in the pocket, ready for use. Toothpaste can become messy if stored with other things, either by leakage or rupture of the container. Deposits form about the cap. There is a potential to spread disease if several people share a toothpaste tube. It is not easy to dispense in simple, measured unit amounts (often children squeeze out far too much). Issues such as fluoride toxicity suggest that better control is desirable. Fluoride has a relatively low safety margin for toxicity.

A compact personal care kit or pack would be useful for those people who are often away from home, or had a forced change of plans, or who find themselves in a "social emergency" such as when about to meet a desirable person or a business contact when thinking about the possibility "are my teeth dirty? Kits of this type should offer an option of oral disinfectant/ mouth freshener, because some odours can arise on the tongue or pharynx as well as between the teeth.

### PRIOR ART

Single-use packaging for oral/dental hygiene purposes includes: EP 0179166 describing single-use dentifrice, as a pile of dry tablets, packed in foil or paper, DE 4238421 describing paste-like single-use pellets of dentifrice or oral therapy compositions, each having a separate, dry exterior wall of gelatine or the like, and distributed within disposable containers such as provided by a blister pack. The wall is broken down inside the mouth. The purpose was to provide a variety of preparations for trials. US 5213428 describes a single-use, bio-degradable finger toothbrush coated with dehydrated toothpaste. The inventor's co-pending application (WO 02/26078) teaches compositions for capsules receiving specific treatments giving them hardened, dried, or coated walls and associated delivery devices for non-paste, non-powder oral hygiene compositions. Compositions including agar for oral/dental hygiene purposes include: EP 0711544 which describes microcapsules having walls of agar and internal contents, within a toothpaste, GB 750126 describing agar-agar as a stabiliser in chemical combination with sodium carboxymethyl cellulose to make jellies, but not single-dose dentifrice beads, WO03/059302 makes use of agar, preferably chitosan or other compounds having an adsorption affinity to lift a film of plaque from teeth - used within a chewing gum. US 5094839 describes a tooth polish (for use by dentists) including diamond dust, made into a semi-gel (not a semi-solid) with gum tragacanth and agar.

US 5961990 discloses a slow-release cosmetic preparation for use on the skin, made of homogenous agar granules but including a "restraining polymer" separate to the gel and reversibly binding to active ingredients. US 6077544 discloses a slow-release delivery package for pharmaceuticals comprising small spheroids with walls and a core, to be compressed into a solid tablet. DE20014157U provides a dentifrice for children made of tiny capsules which burst on vigorous brushing and release flavours, colours and the like in order to encourage good dental hygiene.

FR2822700 discloses a unit dose dentifrice composition containing a gelling matrix enabling it to retain the active ingredients contained therein, in order to have a long-lasting effect in the oral cavity.

### PROBLEM TO BE SOLVED

To devise improved compositions comprising single-use formulations which are more convenient to carry about, yet are commercially feasible.

Another problem is to devise a composition capable of encouraging children to brush their teeth on a regular basis (even when away from home). An alternative convenient and child-friendly; indeed, use-encouraging preparation may be of considerable help in terms of paediatric dentistry and minimisation of public health problems and community costs in later life.

### OBJECT

It is an object of this invention to provide improved oral or dental hygiene compositions or at least to provide the public with a useful choice.

### DEFINITIONS

**BEAD**: We use the term "bead" to describe a soft semi-solid object, which is a unit dose of a novel dental composition, which serves the function of a dentifrice PERSONAL CARE composition. Beads may be supplied in bulk or may be sold within an individual container, including a tape or blister pack. Similies for "beads" are unit doses, lumps, pills, boluses, balls, or tablets.

**DENTIFRICE:** "A substance for cleansing the teeth". Toothpaste is one kind of dentifrice.

**DENTAL ABRASIVE:** Known abrasives used in dentifrice formulations include finely ground insoluble inorganic salts, such as calcium carbonate, dibasic and tribasic calcium phosphate, calcium sulphate, insoluble sodium metaphosphate, hydrated aluminium oxide, and magnesium carbonates and phosphates. Silica and silica xerogels are also used in clear toothpastes. Some toothpastes have no abrasive.

**FOOD GRADE:** a generally defined grade of a non-toxic material which is fully acceptable, particularly with respect to appropriate regulations, for oral intake by people. "GRAS" or "generally regarded as safe" is one category of a food grade product.

**GEL:** "A semi-solid mass, capable of deformation by heat or pressure" 2. "A non-flowing, adhesive mass exhibiting strong cohesive forces having low shear strength". 3. "A colloid in a more solid form than a sol".

**GELLING AGENT:** The molecular structure of a gel is based on a framework of long, variably cross-linked or tangled filaments of a gelling agent, for supporting or containing other molecules or particles. These compounds which are typically polysaccharides or similar, are inherently not easy to characterise by standard chemical methods. Example gelling agents include the gums (see below) and some names are: cellulose ethers, guar, guar derivatives, locust bean gum, psyllium, gum arabic, gum tragacanth, carrageenan, agar, algin, xanthan, scleroglucan, dextran, pectin, starch, chitin, and chitosan. Animal-based gelling agents include gelatin or the like, made from a reconstituted collagen extract. Synthetic agents include those based on a modified natural linear polymer or a synthesised polymer. Naturally occurring seaweed provides gels with a structure based on polysaccharide chains including polymers of D-mannuronic acid or L-guluronic acid (example: alginic acid and its salts), or sulphated galactans (examples include agar, porphyran, furcellaran, and kappa-, iota- and gamma-carrageenan), or combinations, analogues or derivatives thereof. Example food grade structural gels comprise mixtures including one or more of: gelatine, agar or carrageenan as previously described in this section, locust bean gum, guar gum (guaran), locust bean gum, pectin, and starches including corn starch.

**GUM:** A polymeric substance which, in an appropriate solvent or swelling agent, forms highly viscous dispersions or gels at low dry substance content. (For the present application, water-soluble gums are of interest. These include: seed gums, tuber and root gums, seaweed extracts, plant extracts, exudate gums, gums made by microbial fermentation, and derived gums).

**HUMECTANT:** A hygroscopic water-retaining agent which remains moist in air having at least a medium level of humidity. The humectant stops a product including it from drying out, assuming the absence of extreme conditions. Examples include sorbitol, glycerol and some glycols, including propylene glycol and polyethylene glycol.

**SEMI-SOLID:** A descriptor for hardness of a material. A semi-solid material is easily deformed by an applied force, yet retains a predetermined shape on removal of the force unless deformation exceeds a limit. The force of gravity is less than the limit.

**STANDARD CONDITIONS:** An atmosphere of air having a relative humidity of 65%, a temperature of 20 deg Celsius, and a pressure of about 1000 millibars.

**TOOTHPASTE:** A dentifrice in semi-liquid form, including thickeners and viscosity modifiers which serve in part to retain the composition on the teeth and brush during use.

### STATEMENT OF INVENTION

The invention provides a semi-solid homogenous and non-encapsulated gel bead for use in single-dose form in personal dental care, said bead comprising pharmaceutically acceptable active ingredients characteristic of a dentifrice intimately mixed with at least one gelling agent; the at least one gelling agent providing a gel framework comprising sufficient containment means for the active ingredients during storage; the gel framework being capable of breaking apart when the bead is forcibly disrupted by a person and making the active ingredients available for use in a personal dental care procedure; wherein said bead is for use with an application tool including means for contacting a plurality of surfaces of the person's teeth so that after disruption of a bead and release of the active ingredients adjacent to the teeth, use of the tool promotes cleaning of the teeth; and wherein the gelling agent includes agar at a concentration in the range of from 0.4 to 1.1 percent by weight.

The agar gelling agent is present at a concentration at least high enough to cause the composition to remain in a semi-solid state when held at a storage temperature such as below about 30 degrees Celsius, the concentration at the same time being sufficiently low to permit the semi-solid composition to break apart and render the at least one active ingredient available when disrupted by the person.

More preferably the composition exhibits a softening temperature of not less than about 40 degrees Celsius (so that it retains its integrity in a shirt pocket or closed motorcar).

The composition is dimensionally stable under standard conditions, yet is soft enough to permit being fully disrupted within the mouth, during use.

The gelling agent comprises agar at a concentration of from 0.4 to 1.1 percent by weight of the bead.

The composition includes active ingredients capable in use of supporting a dental cleaning procedure on the person's teeth, such as an effective amount of at least one of a surface-active agent (with a foam stabiliser, preferably), and a dentally acceptable abrasive. Optionally, the ingredients include a thickener, a humectant (glycerine), a source of enamel-strengthening material such as fluoride ions, one or more colouring ingredients, and one or more flavouring ingredients.

In a subsidiary aspect, components of the semi-solid composition are substantially as given in Example 1 in the accompanying specification.

Preferably, the bead has a mass in the range of from 0.1 to 2.5 grams, so that a single bead is capable of providing sufficient active ingredients for a single dental care procedure. More preferably, each bead has a weight of 0.4 gram to 1 gram, still more preferably 0.5 to 0.75 grams.

Optionally each bead has a shape adapted to be held by the bristles of a tooth brush.

Optionally the external surfaces of each bead are dusted with a food grade powder in order to maintain separation if stored in contact. Preferably the food grade powder is corn starch.

In a further aspect the invention provides a pack for use in dental care, including at least one bead of a semi-solid gel as previously described in this section, wherein the at least one bead is provided within a compartment.

In a subsidiary aspect, the pack further includes an application tool including means for mechanically agitating or rubbing surfaces of the person's teeth when in use so that after ingestion and disruption, followed by mechanical agitation, deposits upon the teeth may be reduced. The tool may be a brush or a fingercot.

In another subsidiary aspect, the pack includes the tool and at least one bead of a semi-solid composition held within corresponding compartments.

In yet another subsidiary aspect, the compartments comprise depressions made in a deformable plastics sheet, provided beneath a covering, as for blister packs.

In a further related aspect the invention provides a pack for use in oral care, including at least one bead of a semi-solid composition as previously described in this section, wherein the pack includes at least one bead provided within a compartment and wherein the active ingredients of the semi-solid composition include a pharmaceutically acceptable disinfectant material, so that after ingestion and disruption within the mouth, the disinfectant agent causes any odour from the person's mouth or pharynx to be reduced.

The invention also provides a method of manufacture of beads of the invention, which method includes the steps of mixing water with at least one of the pharmaceutically acceptable active ingredients together with the at least one gelling agent at a temperature sufficient to melt the gelling agent, then cooling the mixture to a lower temperature at which composition is still melted, then optionally of adding and mixing at least one further active ingredient, then of expelling the mixture from an orifice.

The method of manufacture of the beads may include the steps of mixing the components in a defined order and at a defined temperature sufficient to melt the gelling agent, optionally of adding flavouring when the mixture is at a lower temperature, then of expelling the mixture from a nozzle, then of cooling the mixture and separating the now semi-solid mixture into beads each of a desired mass, with a desired shape.

The method may include the steps of mixing the components in a defined order, and at a defined temperature sufficient to melt the gelling agent, meanwhile supplying a sheet of material deformed so as to include a plurality of depressions, then of placing each depression beneath a nozzle, then of expelling a controlled amount of the mixture from the nozzle into each depression, then of cooling the expelled mixture so that the mixture solidifies within the or each depression, and then covering the loaded sheet of a material, thereby covering the or each depression with a cover.

The invention also provides a method for assembling a dental care pack as previously described in this section, wherein the method includes the steps of filling one or more depressions formed in a sheet of deformable material as previously described in this section with at least one

selected composition and optionally placing a tool in a further depression, thereby creating a self-contained blister pack.

The invention further provides a complete kit for personal application of dental hygiene, including beads of a dental hygiene composition, dispensing means therefor, and, in addition, brush application means, which kit is provided for sale as a set of items or separately.

Preferably the invention provides means for storing and dispensing beads of a composition as previously described in this section, wherein the means comprises a toothbrush including at least one resealable cavity each capable of storing a plurality of discrete beads, so that the consumable beads and the brush application means for applying the composition to one's teeth are kept together.

In a related aspect, the toothbrush includes two resealable cavities, so that at least one cavity is available for storage of an oral hygiene commodity other than a dental hygiene composition.

The invention also provides a pack for storage of a plurality of beads according to the invention, each held within a compartment along an elongated tape.

Preferably the tape is provided with frangible sections so that compartments can be separated from one another.

For example the invention provides a continuous strip of individually wrapped beads, able to be torn off at a requisite number, offered free or for sale by vending machine, for dispensary, school or hospital use, so that the user can tear open a single portion for single use, the remainder being kept in an hygienic condition until required.

In a related aspect the tape containing beads is made available for personal use.

In another related aspect the tape containing beads within compartments is placed in a dispenser and dispensed on a compartment-by-compartment basis from the dispenser for personal use.

Preferably each dispensed bead remains sealed within each compartment until removed by the person.

In a related aspect the system for delivery of personal hygiene (including beads and application means (such as a toothbrush)) is provided as a set of items at a point of sale, in a readily portable format for use anywhere, such as at home, in an office, and might be provided through a dispensing system for domestic or similar use.

### DRAWINGS

- Fig 1a:: shows a machine for forming blister packs loaded with cast in-situ beads of a composition according to the invention, drawn as a cross-sectional diagram.
- Fig 1b:: shows example loaded blisters in sectional and face view.
- Fig 2:: shows example beads, some in relation to brush heads.
- Fig 3:: is a prior-art bead (2) having a distinct capsule (1). (Krass, DE 423 8421)
- Fig 4:: shows a single-width tape carrying blisters each holding one bead, for use in a dispenser.
- Fig 5:: shows a dispenser for a variety of tapes.
- Fig 6:: shows a blister pack with a toothbrush and five beads.
- Fig 7:: shows a blister pack with sixty beads - a bulk pack.
- Fig 8:: shows a prior-art toothbrush with a bead compartment (Alexander, WO 02/26078)

### INTRODUCTION

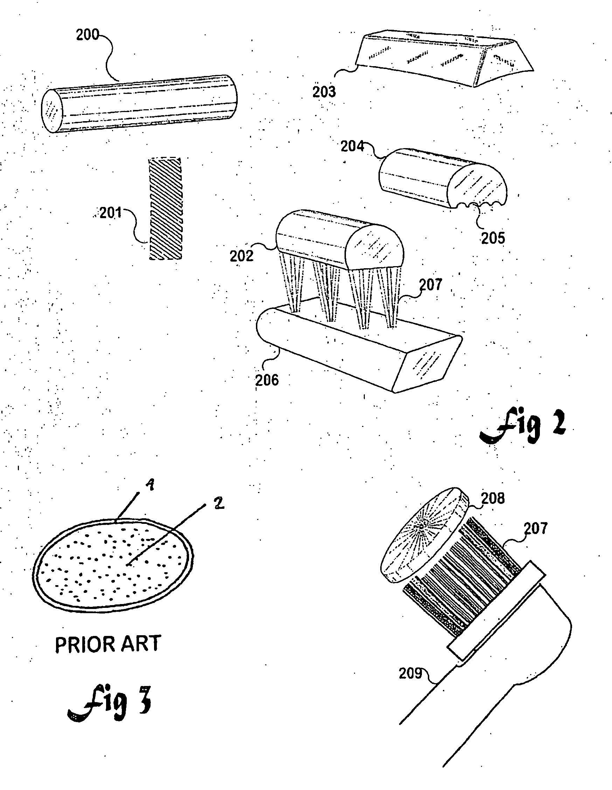

This invention provides a personal hygiene preparation in which a dentifrice including pharmaceutically acceptable active ingredients is made semi-solid by means of agar as a gelling agent and is provided as single-use portions, which are called "beads" (examples: 200 - 204 and 208 in Fig 2). Beads described here lack a separate shell structure such as a gelatine capsule (as used in Krass, see Fig 3) or a dried-out wall. The composition is substantially homogenous (as in cross-section 201 of bead 200). Preferred humectants and use of standard conditions during storage avoid subsequent drying of outer surfaces. There is no adverse "mouth feel" from undissolved fragments of capsule, and no wrapping to be disposed of. The beads do not require containment within a bounded enclosure such as a toothpaste tube. The beads are too solid to be extrudable from a toothpaste tube, if at or below a storage temperature.

The invention could be regarded as employing a "diffuse container" - a gel framework - instead of a physical container (bottle, tube, etc) for the storage of a personal care formulation.

In this invention, one or a mixture of gelling agents used in sufficient concentration allows individual beads to be formed by methods such as casting or extrusion into a cool environment at the time of manufacture. Although the semi-solid beads retain their shapes during extended periods of storage, they will be completely broken apart by the forces involved in brushing teeth, pressing, or biting. Beads maintain individual integrity during storage, even if in contact, and preferred compositions have substantially no syneresis (exudation) during storage. Prior-art gel toothpastes are viscous liquids, not solids. Each bead of a dentifrice composition typically weighs about 0.5 grams, providing about the right amount for a single brushing. (This quantity is a non-limiting example only). Fig 2 shows typical beads in conjunction with appropriate toothbrush heads (206, 209).

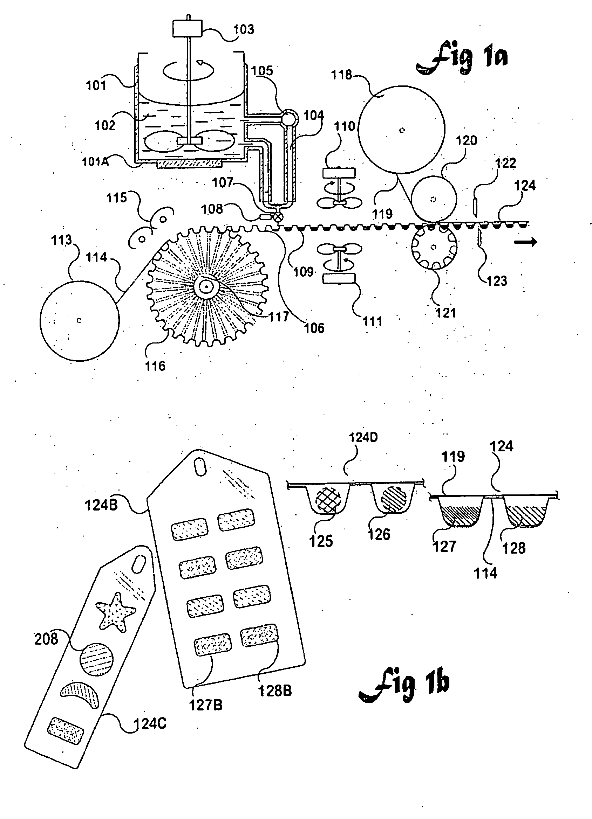

The invention facilitates manufacture of beads of many flavours, colours, shapes, or sizes. Within limits, a different combination of colour, flavour, and/or shape can be selected every time the child brushes his or her teeth, in order to encourage the process by making a game or offering choices. (Blister pack 124C in Fig 1 B exemplifies a child's choice). In order to appeal to children, these features may be exploited in order to make oral hygiene more interesting, even a pleasure, and regular brushings preferably become a self-motivated task. Ordinary toothpaste in a tube provides the same material time after time until the tube runs out - a very long time to a child. This variety of visually different yet functionally similar beads is intended to promote the frequency of voluntary cleaning of teeth (offering a choice being a well-known subterfuge when causing children to comply with their parents' requests). Differing sizes, shapes, colours, flavours (and other additives) may be made for different markets or to suit particular toothbrushes.

Packaging may be as bulk beads, in a single container. A presently preferred method for packing and presentation, still cheaper than the use of tubes to hold standard toothpaste, uses the well-known "blister pack" as for singly wrapped tablets and capsules ready for use, within "bubbles" of a moulded plastics material held against a stiffer backing of card. Versions with an adherent plastics or metallic foil sheet create cavities that are effectively impervious to moisture loss. Opening any one does not affect the remainder. Individual lumps of dental composition may be either loosely contained within bubbles (124D), made as in, for example, Example 1, or may be adherent to the interior of the bubble (124), as per Example 3. The dental hygiene composition is provided as separate, semi-rigid and non-sticky lumps (the beads) in the form of a free-flowing material, no longer needing the special containment provided by a toothpaste tube. The resulting beads are easily selected, handled, and used as required because the agar component tends to ensure that each bead holds together. In use, one or perhaps more beads are selected for use, removed from a container or dispenser and mechanically disrupted within the mouth perhaps by finger pressure, pressing the bead on to the bristles of a brush, or against the teeth, or by being bitten. After that step, a conventional toothbrush (which may be electric) is used in the normal manner.

Figs 1B and 2 show a range of shapes. Cylindrical beads 200 are easy to extrude from a nozzle and then cut into beads of a desired weight for packing in a container, while the "D" section version (204) may likewise be made by casting from a shaped nozzle. A flat face as in 203, 205, and 402 may enhance attachment to the bristles 207 of a brush 206 prior to use. Optionally the bristle face 205 may be made irregular so that initial holding is made easier. (Other shaped nozzles can be used to extrude moons, stars, and other shapes if required). The solid shape (203) is an example obtained by in-situ casting into the blister pack (see Example 3) so that each bead has a controlled shape.

Beads can be made by extrusion then slicing, or casting, in a discoidal shape 208 to better fit the circular array of bristles on the head of an electric toothbrush 209 (e.g. a "Braun"). A person can decant some beads from a large container into a small container as for example when travelling. The "small container" may for example be constructed within a toothbrush handle. Loose beads (200) made as above can be sold with or inside the applicant's prior-art toothbrush (Fig 10) having a cover 1000, bristles 1001, a handle 1002, and a lid 1003 opening into a compartment for beads. (This prior application envisaged capsules, not homogenous beads).

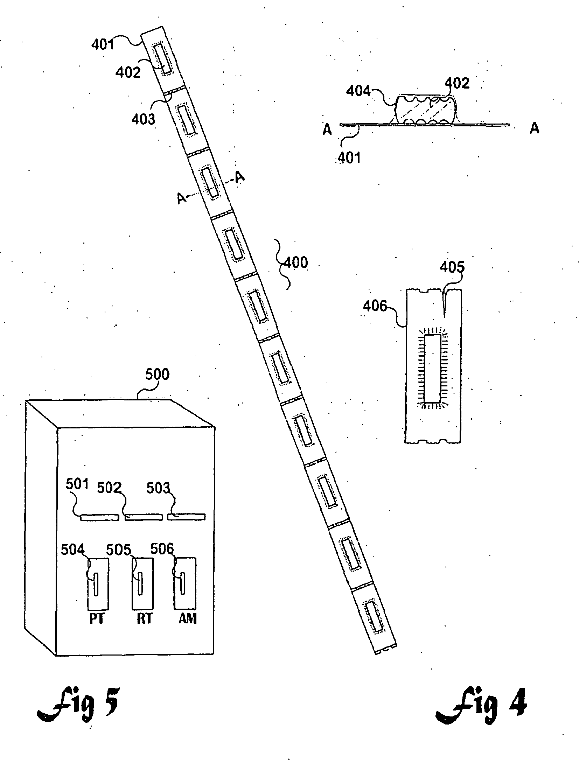

Use of tapes, each compartment along the tape holding a separate bead, is an alternative to loose packing or blister packs. Fig 4 illustrates part of an elongated tape 400 holding a plurality of individual beads 402 each in a perforation-bounded (403) rectangle of a substrate such as paper 401, suitable for rolling into a reel and dispensing from a machine (Fig 5). Section A-A is shown to the right. Note that this section shows an extruded bead in section, inside a blister, although the bead could have been cast in the blister. A tearable opening means is shown at 405 in the single rectangle 406 (below). A film of a plastics material 404 protects each bead until used. This type of product may be made in a many-bead width and then sliced into single or dual (not shown) strips. Fig 5 shows a wall-mounted dispensing machine having outlets 501, 502, and 503 for a variety of beads, each one selectable (in this example) by placing a coin or token in a related slot (504, 505, or 506). This would be suitable for use in an ablutions area such as in a boarding school, office, restaurant, or campsite.

### REFERENCE EXAMPLE 1 Gelatine

Gelatine is a gelling agent made out of animal by-products. It has a relatively low melting point which affects its suitability.

| ***Component*** | **% *w*/*w*** |
|---|---|
| Hot water | q.s. |
| Gelatine | 4 |
| Glycerine | 20 |
| Texapon OC-N | 1 |
| Synthecol CAB | 2 |
| Sodium chloride (V41 salt) | 1.3 |
| Sodium saccharin | 0.1 |
| Dicalcium phosphate dihydrate | 50 |
| Sodium fluorophosphate | 0.7 |
| Sodium dihydrogen citrate | 0.5 |

| Flavour Version a: | |
|---|---|
| Peppermint oil | 0.6 |
| Menthol | 0.1-0.2 |

| Flavour Version b: | |
|---|---|
| Orange oil | 0.7 |
| Tartrazine (dye) | 0.1 |

| Flavour Version c: | |
|---|---|
| Strawberry flavour | 0.7 |
| Ponceau Red 0.2% (dye) | 0.1 |
| | 100 |

Flavour versions shown are examples of possible sets of flavours and corresponding colours.

There is little or no chemical reaction between any of the individual components of this composition. Gelatine is based on a reconstituted collagen extract and is widely available as a foodstuff. Glycerine (glycerol) serves as a humectant. Texapon (R) OC-N (Henkel NZ Ltd) is a commercial brand of sodium lauryl sulphate (an anionic surface-active agent) having minimal taste or flavour of its own. Synthecol CAB (coco amido propyl betaine; Chemcolour Industries (NZ) Ltd) is a commercial brand of a foam stabiliser/thickener having minimal taste or flavour of its own. Supplied as a liquid having 30% active concentration. V41 salt is a vacuum-dried sodium chloride (Dominion Salt, NZ). Sodium saccharin (Bronson & Jacobs) is a sweetening agent. Dicalcium phosphate dihydrate (Victor DF) (Rhodia Food, Cranbury NJ USA) is a substantially insoluble salt, used as an dentifrice (abrasive). The fineness of the powder is such that 99.5% will pass through a 325 mesh. Sodium monofluorophosphate is a source of fluoride ions generally used for local hardening of the enamel, that is, as a source of tooth strengthening ions. It is preferred over sodium fluoride for safety reasons in particular. Calcium glycerophosphate (1-4 %) is optionally added; it may act as a reservoir of calcium ions for the teeth and/or as a "buffer" for fluoride ions. Preferred dyes are (a) food-grade and (b) fade resistant, as from sunlight. A tendency we have noted (when beads of different colours are stored together) for soluble dyes to diffuse into adjacent beads and blending with other colours suggests that adsorbing a colour onto the abrasive particles may be a preferable colouring method. A laking pigment (adsorbed onto a substrate such as aluminium oxide powder) may be preferred.

### Manufacture:

1. Add gelatine, glycerine, Texapon OC-N, Synthecol CAB, salt, and sodium saccharin to a jacketed vessel and mix.
2. Add hot water and mix while holding the temperature.
3. Add dicalcium phosphate and sodium fluorophosphate and mix to a smooth white liquid.
4. Let the temperature drop to about 65 deg C while mixing.
5. Dissolve the menthol in peppermint oil. (or flavour/ colour options (b) and (c) if selected).
6. Add the flavour/colour option mix and the citric acid when temperature is less than 50 deg C and mix.
7. Pump mixture through a chilled 6.5 mm ID pipe onto a conveyer belt and cut into approximately 10 mm lengths. (200 items).
8. Dust each bead lightly with cornflour.
9. Pack into an airtight container.

Beads were opaque cylinders, white or of a pastel colour if a dye was added.

These results refer to peppermint/menthol versions. In terms of shelf life and related storage, we found that gelatine-based beads were not able to hold their shape well, especially at raised temperatures, in accordance with the known properties of gels based on gelatine.

Sample beads were placed in direct sunlight on a window sill for 3 hours, whereupon the outside shell crusted marginally but remained pliable and usable. The inventor left sample beads exposed in a steamy bathroom for 3 hours. They retained their shape and did not get tacky or mutually adherent, and remained usable.

Samples were placed in direct sunlight, outside in a clear plastic bag. They melted and lost shape under temperatures exceeding 30 deg C. They were usable when cooled but had lost their shape. Samples placed in a plastic bag and left on the back floor (in shadow) of a black car in direct sunlight, at a temperature of at least 28 deg C softened but did not lose shape; they could be handled carefully and, not becoming tacky through syneresis, could be used. If stored together in a canister it may under adverse conditions be difficult to extract an individual bead for use, although dusting with a powder such as cornflour would help.

The storage characteristics of this version may not meet commercial requirements because storage temperatures of over 30 deg C or outside "standard conditions" could result in loss of shape and integrity. To raise the concentration of gelatin beyond about 5% renders the beads less attractive to users in terms of "mouth feel" and less disintegration during use. There may be other ways to overcome the low-temperature melting problem, such as the use of a gelatine extracted under different conditions, or use of a mixture including at least one other gelling substance. At the preferred concentration of glycerol, there was some reversible tendency for the periphery of each bead to lose water and harden slightly when in an atmosphere of low humidity.

As an alternative, a jube type of bead as above would have at least 5% by weight of gelatine.

### REFERENCE EXAMPLE 2: No abrasive

| ***Component*** | **% *w*/*w*** |
|---|---|
| Hot water | q.s. |
| Gelatine | 8 |
| Glycerine | 40 |
| Texapon OC-N | 2 |
| Synthecol CAB | 4 |
| Sodium chloride (V41 salt) | 2.6 |
| Sodium saccharin | 0.2 |
| Sodium fluorophosphate | 1.4 |
| Sodium dihydrogen citrate | 1 |
| Peppermint oil | 2 |
| Menthol | 0.8 |
| | 100 |

Some people consider that the abrasive/dentifrice component of toothpaste, used to physically attack plaque, is not needed especially with use of electric toothbrushes and hence this option deletes the abrasive. Half-size (0.25 g) beads may be sufficient to fulfil the functions of non-abrasive dentifrice in which case 100 g of the above mixture is sufficient for about 400 beads. Each bead would be translucent because of deletion of the opaque abrasive.

### REFERENCE EXAMPLE 3: Clear bead

| **Component** | **% w/w** |
|---|---|
| Hot water | q.s. |
| Gelatine | 4 |
| Glycerine | 20 |
| Texapon OC-N | 1 |
| Synthecol CAB | 2 |
| Sodium chloride (V41 salt) | 1.3 |
| Sodium saccharin | 0.1 |
| Hydrated silica (dental abrasive) | 30 approx. |
| Sodium fluorophosphate | 0.7 |
| Peppermint oil | 1 |
| Menthol | 0.4 |
| | 100 |

The refractive index of hydrated silica is similar to that of the matrix in which it is suspended. The appearance, shape, and colour of the resulting clear bead may be varied in order to appeal to different sectors of the population.

### EXAMPLE 1: Agar.

Agar offers considerable advantages as a gelling agent over gelatine, including better temperature characteristics, marked hysteresis between melting and setting, low syneresis, and is not of animal origin. Use of trial concentrations of agar in a dentifrice composition showed that no adverse reactions occurred.

| **Component** | **% w/w** |
|---|---|
| Boiling water | 32.1 |
| Agar (Note A) | 0.8 |
| Glycerine | 15 |
| Texapon OC-N | 1 |
| Synthecol CAB | 2 |
| Sodium chloride (V41 salt) | 1.5 |
| Sodium saccharin | 0.1 |
| Dicalcium phosphate dihydrate | 45 |
| Sodium fluorophosphate | 0.7 |
| Peppermint oil No. 1 | 1 |
| Menthol | 0.4 |
| Citric acid | 0.4 |
| | 100% |

Agar (CAS 9002-18-0) was obtained from Coastal Biologicals, Auckland, New Zealand as "standard agar" type MG. Gel strength of a 1.5 % gel is 1000 g/cm². Melting point is 85-95 deg C. It should be emphasised that properties of a New Zealand agar may be unlike those of an agar gathered from another coastline and therefore optimised concentrations for other kinds of agar may differ from those given herein. The scope of the invention includes synthetic agar or analogues made by (for example) recombinant micro-organisms such as yeasts or bacteria.

Variations in the concentration of this agar were tested. Values of around 0.2 % - 0.5 % give beads which are too soft and friable. Above about 1.2 % gives beads which are too hard and which have poor mouth feel - they do not break up easily when in use and they are difficult to hold on the brush. It would appear that concentrations of about 0.7 to 0.9 % are preferable, for this type of agar if used to make free beads. (Use in Example 3 type blister packs may allow lower concentrations of agar). Agar may be mixed with more widely available gel materials (such as gelatin) may be preferred. The quantity of agar used is preferably minimised for cost reasons.

Other flavouring and colouring agents were substituted for the peppermint oil/menthol combination shows here, in some samples, as for Reference Example 1.

### MANUFACTURE:

1. Add agar, glycerine, Texapon OC-N (wetting agent), Synthecol CAB, salt, and Na saccharin to a jacketed vessel and mix.
2. Add boiling water q. s. and mix while raising the temperature to 95-100 deg C in order to fully dissolve the agar.
3. Add dicalcium phosphate and sodium fluorophosphate, and mix to a smooth white liquid.
4. Let the temperature drop to 65 deg C while mixing.
5. Dissolve the menthol in peppermint oil.
6. Add the menthol/peppermint mix and the citric acid when the temperature is less than about 50 deg C and mix.
7. Pump mixture through a chilled 6.5 mm ID pipe onto a conveyer belt and cut into approximately 10 mm lengths. (200 items). (The mixture sets at about 35-40 deg C).
8. Pack into an airtight container.

Appearance: a crisp white gel with an oily surface. (An inherent property of agar is that it provides a gel which will not re-melt unless heated to well above the setting/gelling temperature, useful for storage or carriage at elevated temperatures). The pH of a 10% slurry in water = 5.95. Dimensions: An about 6.5 mm diameter, 10 mm long cylinder weighs about 0.5 grams.

It will be evident to one of skill that the method described above is capable of application on an industrial scale; whether carried out in a batch mode or in a continuous mode. (See Example 3) Reliance on temperature to cause setting is usually more convenient than reliance on completion of a chemical reaction. It will also be evident to one of skill that a variety of ways to finally "cast" or otherwise shape each bead are available from various fields of industrial arts. For example the mixture, while above its gelling temperature, may be dripped or extruded on to a cold belt, may be injected into or onto a mould, smeared over a plate or belt having shaped depressions, or extruded from a shaped aperture, so that the mixture gels and assumes the shape in which it is constrained at the time.

We found that beads made according to this method had acceptable mouth feel, and retained this status even after storage for extended periods of some months, and at elevated temperatures. No bacterial or fungal growth has occurred. Some beads were dusted with cornflour as an anti-stickiness treatment, but all remained as separate beads regardless. There was a tendency for components to diffuse between beads in contact, as shown by migration of colouring agents. Beads are therefore suitable for storage in compartments built into the handles of toothbrushes.

Informal, confidential tests for acceptability and effectiveness were carried out on about 20 people aged from 6 to 82 years. Their responses indicated that the product was quite acceptable. A surprising finding was that some people preferred beads over toothpaste. It was easier to rinse and clear the material from the mouth. This may be because toothpaste includes thickeners.

Tests of the above Examples have concentrated on two forms of long-chain gelling agent; gelatine and agar, in the first instance. Agar has a higher melting point which would withstand common environmental exposures such as inside pockets or inside cars. If the beads melt and re-solidify they lose their shape but their properties are not altered.

### EXAMPLE 2 Blister pack - loosely packed beads.

A method for dispensing the invention exploits the "blister pack" as widely used for presenting individual tablets ready for use, within "bubbles" of a moulded clear plastics material held against a backing sheet. A "blister pack" has a shaped array of depressions each capable of holding an object, sealed off at a surface by a backing card or sheet. Depressions, usually formed within a thermoplastic plastics film by vacuum forming the film when hot against a die, may be of any desired shape. Versions backed with an adherent plastics or metallic foil sheet create relatively moisture-impervious cavities. In those versions the backing card or sheet is punctured or torn from behind any one cavity in order to expose the contents. In other versions the backing sheet may slide sideways within guides as is known in the art in order to release the contents of a cavity. Simple versions of that option do not provide hermetic sealing but the beads with humectant are in any case adapted for stability in a standard atmosphere or most habitable atmospheres. Reclosable blister packs are well-known, and may be particularly applicable.

Preferably the material of the blister pack is bio-degradable, such as a celluloid or a selected plastics material.

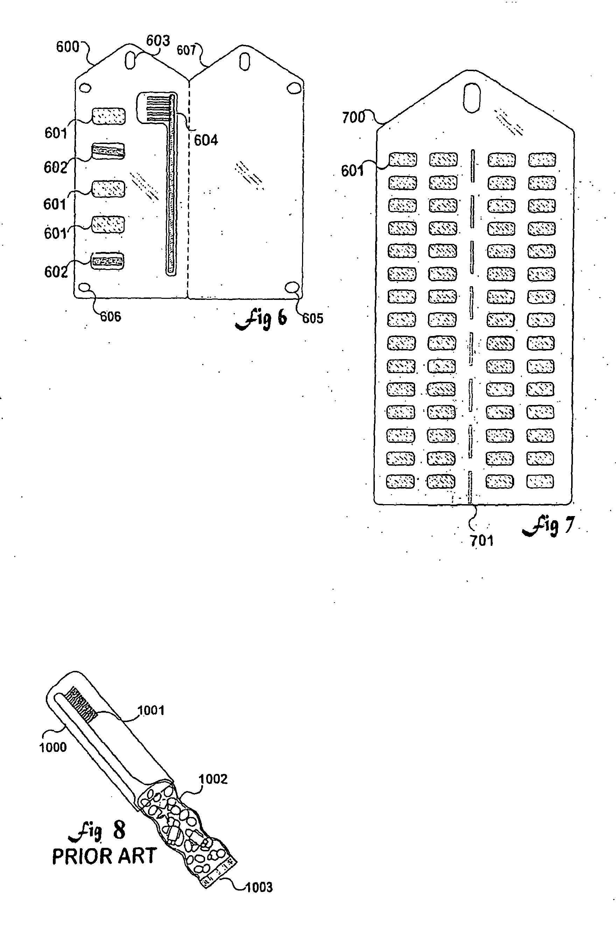

Individual lumps of dental composition may be either loosely contained within bubbles, having been made by for example as in Example 1, or may be adherent to the interior of the bubble (Example 3). See Fig 1b, the left side of the cross-section 124, showing loose beads 125, 126 within a blister pack. A blister pack or "dental care kit" may include loose beads together with a disposable brush 604 (see Fig 6). Another "dental care kit" is a prior-art toothbrush (Fig 8) having a cover 1000, bristles 1001, a handle 1002, and a lid 1003 opening into a compartment for beads. Colour coding allows specific beads to be used for oral or dental care.

### EXAMPLE 3 Beads cast in-situ within blister-pack.

This Example is illustrated schematically in section in Fig 1 a. It is a production line (parts 110-123) for making and loading blister packs (124), operated in conjunction with at least one subsidiary production line (parts 101-108) for preparing at least one composition (or several compositions in parallel) including a gelling agent according to this invention. The composition 102 held in container 101, having a hot-water jacket 101A and stirred by stirrer 103 is a molten gel, to be delivered through dispensing means (recirculating pump 105 and jacketed recirculating pipe 104, the pipe passing heated valve 107 controlled by actuator 108) which can deliver composition into blisters or containers (106; empty, 109; filled) which after being filled are then cooled such as by cooling fans 110 and 111 (or water sprays, or a chilled bed or conveying means), and sealed. The resulting beads of composition as sold to a user within a blister pack 124, 124B, 124C comprises a cast mass of a controlled amount, cast within its container, and lying in contact with the interior of the blister. Preferably the cast mass sets in the bottom of the blister as shown in cross-sections 127 and 128; also 127B and 128B in Fig 1 b. Other parts of the production line include a modified version of the usual blister pack process. 113 is a supply reel of thermoplastic film, which film 114 passes over a rotating, cylindrical array of dies 116. The film is pre-heated for example by radiant heaters 115 or with hot air in order to soften it for being pulled into the dies by a vacuum, applied through pipes leading into a vacuum manifold through a slide valve 117. The shaped film bearing depressions to be filled leaves the cylinder and is filled at the hot valve 107 as previously described in this section. The beads are sealed in for example by hot-pressing a layer of adhesive-coated foil 119 supplied from reel 118 between pressure rollers 120 and 121. Finally the strip is cut with knives 122, 123 into usable portions 124, 124B, 124C. (The arrow shows the direction of motion).

One such usable portion is the blister pack 124B of Fig 1b, in which two kinds of bead 127B, 128B were placed by duplicated subsidiary production lines (101-108) for supplying the composition. Only one production line is shown in Fig 1. The backing foil may carry indicia such as brands, identification of components of the composition, use-by dates, and country of manufacture. Pack 124C is a children's pack in which a variety of shapes of cast in-situ bead (eg 208) are created by suitable shaping of the wheel 116 carrying dies, and attractive combinations of colorants and flavours within the beads themselves. Blister packs display the shapes and colours clearly. A child can select a crescent moon one day, a star another day, and so on - as shown in Fig 3. Many other series of shapes will be apparent to a skilled reader.

Blister pack shapes and sizes may be selected so that one or more packs are easily loaded into a compartment within a handle of a re-usable toothbrush (of the type shown in prior-art Fig 10). Advantages of cast in-situ beads over manual or robotic placement of previously cast beads in each blister include that the manufacturing is more hygienic and more easily automated. Only "tools" such as brushes, if desired, need to be physically inserted. A user may separately open any one blister usually by puncturing the foil from the rear and expelling the contents, when the cohesive nature of the agar causes the bead to part from the film as a single unit when opened. A person could press on the front of the pack (the depression side) in order to free the cast in-situ bead. The person would press a toothbrush head into the bead within the opened blister from the rear and hygienically and in a sterile manner push the bead (held by the plastic) straight on to the brush. Release from the film may be aided by lightly pre-coating the inner surface of the front of the pack with a slippery plastics or coating material, as is known in the relevant art.

Quality control at manufacture is simple, largely because the preferred blister packs have transparent fronts and an optical device such as a camera coupled to image analysis apparatus can quickly ensure that each filled blister satisfies acceptance criteria such as colour, density, and dimensions. The agar content of the composition may be reduced in comparison to that of free-flow (bulk) beads, while still considering factors such as the re-melting temperature. Dye and flavour diffusion, sometimes noted when free beads are stored in contact, cannot occur.

As a development over Fig 1b, a pack (600 in Fig 6) may include a toothbrush 604 and is hence a ready-to-go kit. Disposable toothbrushes for inclusion in this pack are widely available at low cost for use by manufacturers. This example includes three beads (cast in-situ) of dentifrice/toothpaste 601 (which might have differing flavours), and two free beads of a mouth freshener 602. This pack is likely to be a convenient combination for people away from their homes. The cast in-situ depressions may later be replaced by the user from a stock of free beads as shown in Fig 7 or from a container of loose beads. This version of a blister pack includes a reclosable back 607 hinged along the dotted line, shown in the open position in Fig 6, and able to be held closed by fasteners 606 fitting into holes 605, so that a person can re-use the same container from time to time. The pack may be displayed for convenient sale on a hook, using hole 603. The blister pack 700 (Fig 7) illustrates a bulk supply pack with a central fold 701. 60 beads as shown would suffice for a month for people who brush twice daily, although current recommendations are for three brushings daily.

A variation in the style of use of beads as replacement for toothpaste was noted. Some people use a dry brushing action at the start then add water and reported that the beads are compatible with this procedure. Therefore the beads with an oral hygiene system (beads, container, and brushing means) could be used without water if necessary - saliva will flow as a result of the action.

### VARIATIONS

Any reasonable combination or number of beads (and tools) may be selected for any purpose-assembled blister pack. With the underlying intention of providing a convenient personal care pack, a comb, a toothbrush, and some beads of dental care products may be put into one blister pack. A complementary blister pack would contain skin care beads (including soap, moisturiser, and sun screen, and shaving soap and aftershave. Some people use electric shavers, others would buy a pack including a disposable blade razor. Another application for a re-closable blister pack (as shown in Fig 6) is for a toothbrush and one or two beads to be included on a daily basis in a lunch box.

Special formulations (such as certified kosher, halal, vegetarian, and vegan) may be made to suit particular needs, or to overcome problems such as particular allergies that some people have.

One might include other known toothpaste/dentifrice components, such as chlorophyll (for example in the form of a water-soluble chlorophyllin - a metal salt - as described for example in GB 720720, at concentrations of around 0.1%). This would also serve as a green colouring agent.

Ordinary toothpaste may be provided in blister packs as single-use units, perhaps with a variety of flavours or the like, thereby securing some of the advantages of the invention, but as a viscous paste would be more difficult to remove for use in the absence of a gel structure giving the contents of the blister pack a cohesive property.

### COMMERCIAL ADVANTAGES

Advantages realised during manufacture of this dental hygiene system include:
1. A bead having no external or capsule is simpler to mix, make, and pack than one having a specific outer capsule or coating and is wholly convertible into an oral, dental or skin care composition when broken up within the mouth by action of teeth and tongue, or with the aid of a toothbrush. There is no unpleasant mouth feel, and no residue to be swallowed or disposed of.
2. Semi-solid beads are simpler to manufacture than toothpaste tubes filled with a viscous paste, especially with the cast-in-blister approach.
3. Preferred processes that employ a gelling agent use a fall of temperature to initiate setting; more convenient than use of a timed chemical reaction (although that advantage does not preclude use of a chemical reaction for some instance.

Inherent advantages of the beads of this invention include.
1. Convenience in storage, handling, and use; the semi-solid material behaves as dry granules until used. The beads are easily stored, decanted, and dispensed, and cannot contaminate a traveller's suitcase in the same way as spilt toothpaste. They travel well and remain hygienic.
2. There is an opportunity to mix shapes, colours and flavours and add some visual attractiveness, especially for children, within the one pack. Ordinary toothpaste tubes provide the same mixture for a long period of time. Children can pick a flavour or a shape, like a star for the evening and a sun for the morning.
3. The single dose nature of beads limits waste, and reduces risks associated with fluoride overdose. (Excess toothpaste cannot be returned to the tube).
4. A single (tape or bead) dispenser may be shared, without hygiene or cross-infection problems, by groups of people (such as a family, or a school camp, trampers/hikers, travellers, or military groups).
5. Tablets or beads of breath freshener, headache pills, soap, moisturiser, sunscreens, or the like can be stored in the same container as beads of dentifrice, assuming helpful colour or shape differentiation. (This is particularly useful for a traveller).
6. The ability to formulate beads without use of animal by-products suits some people.

Advantages to a user of the dental hygiene system (in particular) include:
1. Beads of the invention are highly compatible with use of portable toothbrushes. Prior art for portable toothbrushes involves use of standard semi-liquid toothpastes which are difficult to handle, keep moist, and contain in place.
2.The invention includes most of the either already FDA-acceptable or "GRAS" (generally regarded as safe) conventional components of known toothpastes but held in a different physical form (a semi-solid gel).
3. Each member of a family may use the flavour and type of dentifrice that they prefer, an advantage when teaching children to clean their teeth voluntarily.
4. Some triallists liked the ease of rinsing and clearing the material from the mouth after use. This may reflect an absence of thickeners..
5. A bead of dentifrice in a blister pack can be securely put on the bristles of a brush in a sterile manner, without touching the bead.
6. The invention enhances existing programmes of tooth care and dental hygiene, especially those for children, where the variety of beads and the control of the amount in each is likely to assist in voluntarily brushing the teeth. Tape dispensers could be used.
7. The dental hygiene system (beads, container, and brushing means) has an enhanced portability because a compact article provides all materials and tools required for cleaning the teeth and freshening the mouth (except water).
8. This easily distributed dental hygiene system based on beads would assist with third world health programmes, in addition to the reduction of cross infection from communicable diseases.
9. The dental hygiene pack is convenient for travelling people, or for holding in reserve in a handbag, briefcase, desk drawer, or car compartment, and for outdoors and military personnel or the like. The pack is designed for a good storage life. The weight and volume of an entire toothpaste tube is an unnecessary extra load and consumer of space.
10. Promotional toothbrushes as used by airlines and hotels for example are more conveniently manufactured and distributed together with this more easily handled dentifrice composition. Kits may be refilled from dispensers or convenient pocket packs.
11. Toothbrush design can be adapted for the storage of a small number of beads within the handle, thereby resulting in a single object for a single function (cleaning the teeth). Even the ubiquitous narrow plastic handled toothbrush can be made with a visible internal cavity and a removable, perhaps resilient resealable plug for accessing a bead as required.
12. A toothbrush having a solid, shaped handle with a cavity for containing beads or blister packs holding beads can be made with a cover for travelling. The cover may become part of the handle when in use, consequently reducing the bulk to be carried.

## Claims

1. A semi-solid homogenous and non-encapsulated gel bead for use in single-dose form in personal dental care, said bead comprising pharmaceutically acceptable active ingredients characteristic of a dentifrice intimately mixed with at least one gelling agent; the at least one gelling agent providing a gel framework comprising sufficient containment means for the active ingredients during storage; the gel framework being capable of breaking apart when the bead is forcibly disrupted by a person and making the active ingredients available for use in a personal dental care procedure; wherein said bead is for use with an application tool including means for contacting a plurality of surfaces of the person's teeth so that after disruption of a bead and release of the active ingredients adjacent to the teeth, use of the tool promotes cleaning of the teeth; and wherein the gelling agent includes agar at a concentration in the range of from 0.4 to 1.1 percent by weight of the bead.

2. A bead as claimed in claim 1, which has a mass in the range of from 0.1 to 2.5 gram.

3. A bead as claimed in claim 2, which has a mass in the range of 0.4 to 1 gram.

4. A kit or pack for use in dental care, including at least one bead of a semi-solid gel as claimed in claim 1, stored within a compartment within the pack.

5. A kit or pack as claimed in claim 4 further comprising an application tool including means for contacting a plurality of surfaces of the person's teeth, so that after disruption of a bead and release of the at least one active ingredient adjacent to the teeth, use of the tool promotes cleansing of the teeth.

6. A kit or pack as claimed in claim 4, wherein each compartment comprises a depression formed in a deformable sheet and covering means for covering the depression.

7. A method of manufacture of beads as claimed in claim 1, which method includes the steps of mixing water with at least one of the pharmaceutically acceptable active ingredients together with the at least one gelling agent at a temperature sufficient to melt the gelling agent, then cooling the mixture to a lower temperature at which the composition is still melted, then optionally of adding and mixing at least one further active ingredient, then of expelling the mixture from an orifice.

8. A method as claimed in claim 7, wherein, after expulsion from the orifice, the mixture is cooled so as to cause solidification, then the solidified mixture is separated into one or more homogenous beads each having a desired mass.

9. A method as claimed in claim 7, wherein the apparatus used in the manufacture includes means for deposition of a controlled amount of the melted composition from the orifice into at least one of a plurality of depressions formed in a deformable sheet, means to allow the molten composition to solidify, thereby forming at least one cast-in-place bead, and means to enclose the at least one bead by placement of a covering sheet over the depression, so that a pack holding at least one cast-in-place bead is thereby manufactured.

10. A method as claimed in claim 7, wherein the apparatus used in the manufacture includes means for inclusion in the pack or kit of toothbrushes.

## Patentansprüche

1. Halbfeste homogene und unverkapselte Gelperle zur Verwendung in Einzeldosisform in der persönlichen Zahnpflege, wobei die Perle pharmazeutisch verträgliche aktive Bestandteile aufweist, welche kennzeichnend für eine Zahnpaste, welche gründlich mit mindestens einem Geliermittel vermischt ist, sind; wobei das mindestens eine Geliermittel ein Gelgerüst zur Verfügung stellt, welches ausreichende Eindämmungsmittel für die aktiven Bestandteile während der Lagerung aufweist; wobei das Gelgerüst imstande ist auseinanderzubrechen, wenn die Perle durch eine Person gewaltsam zerrissen wird, und die aktiven Bestandteile zur Verwendung in einem persönlichen Zahnpflegeverfahren verfügbar zu machen; wobei die Perle zur Verwendung mit einem Werkzeug einschließlich Mittel zur Kontaktaufnahme mit einer Vielzahl von Oberflächen der Zähne der Person ist, so dass nach Zerreißen einer Perle und Freisetzung der aktiven Bestandteile angrenzend an die Zähne, die Verwendung des Werkzeuges das Reinigen der Zähne fördert; und wobei das Geliermittel Agar in einer Konzentration im Bereich von 0,4 bis 1,1 Gewichtsprozent bezogen auf die Perle einschließt.

2. Perle wie in Anspruch 1 beansprucht, welche eine Masse im Bereich von 0,1 bis 2,5 Gramm hat.

3. Perle wie in Anspruch 2 beansprucht, welche eine Masse im Bereich von 0,4 bis 1 Gramm hat.

4. Kit oder Packung zur Verwendung in der Zahnpflege, welches mindestens eine Perle eines halbfesten Gels wie in Anspruch 1 beansprucht, in einer Abteilung innerhalb der Packung aufbewahrt, einschließt.

5. Kit oder Packung wie in Anspruch 4 beansprucht, welches weiterhin ein Werkzeug einschließlich Mittel zur Kontaktaufnahme mit einer Vielzahl von Oberflächen der Zähne der Person aufweist, so dass nach Zerreißen einer Perle und Freisetzung des mindestens einen aktiven Bestandteiles angrenzend an die Zähne, die Verwendung des Werkzeuges das Reinigen der Zähne fördert.

6. Kit oder Packung wie in Anspruch 4 beansprucht, wobei jede Abteilung eine Vertiefung, welche in einer verformbaren Platte geformt ist, und Abdeckungsmittel zur Abdeckung der Vertiefung aufweist.

7. Verfahren zur Herstellung von Perlen wie in Anspruch 1 beansprucht, wobei das Verfahren die Schritte einschließt, dass Wasser mit mindestens einem der pharmazeutisch verträglichen aktiven Bestandteile zusammen mit dem mindestens einen Geliermittel bei einer Temperatur gemischt wird, die ausreicht, um das Geliermittel zu schmelzen, dann dass die Mischung auf eine niedrigere Temperatur heruntergekühlt wird, bei welcher die Zusammensetzung noch geschmolzen ist, dann dass wahlweise mindestens ein weiterer aktiver Bestandteil hinzugefügt und vermischt wird, dann dass die Mischung aus einer Öffnung herausgestoßen wird.

8. Verfahren wie in Anspruch 7 beansprucht, wobei die Mischung nach Herausstoßen aus der Öffnung so gekühlt wird, dass Verfestigung verursacht wird, dann die verfestigte Mischung in eine oder mehr homogene Perlen, welche jede eine gewünschte Masse hat, aufgeteilt wird.

9. Verfahren wie in Anspruch 7 beansprucht, wobei das Gerät, welches zur Herstellung verwendet wird, Mittel zur Absetzung einer kontrollierten Menge der geschmolzenen Zusammensetzung aus der Öffnung in mindestens eine einer Vielzahl von Vertiefungen, welche in einer verformbaren Platte geformt sind, Mittel, die es der geschmolzenen Zusammensetzung erlauben, sich zu verfestigen, wodurch mindestens eine eingegossene Perle geformt wird, und Mittel, um die mindestens eine Perle durch Platzierung einer Abdeckplatte über die Vertiefung zu umschließen, so dass eine Packung, die mindestens eine eingegossene Perle enthält, **dadurch** hergestellt wird, einschließt.

10. Verfahren wie in Anspruch 7 beansprucht, wobei das Gerät, welches zur Herstellung verwendet wird, Mittel zur Einfügung in die Packung oder das Kit von Zahnbürsten einschließt.

## Revendications

1. Perle de gel semi-solide homogène et non encapsulée, à usage sous forme de dose unique en soins dentaires personnels, ladite perle étant composée de principes actifs pharmaceutiquement compatibles caractéristiques d'un dentifrice, intimement mélangés avec au moins un gélifiant, le/les agents gélifiants constituant une structure en gel comportant des moyens de rétention suffisants, pendant le stockage, pour les principes actifs ; la structure en gel étant apte à se désagréger lorsque la perle est amenée à se rompre par une personne et permettant une utilisation des principes actifs lors d'une action de soins dentaires personnels ; ladite perle étant conçue pour servir à l'aide d'un outil d'application comportant des moyens destinés à venir au contact d'une pluralité de surfaces des dents de la personne afin que, après rupture d'une perle et libération des principes actifs au voisinage immédiat des dents, l'emploi de l'outil contribue au nettoyage des dents ; et le gélifiant contenant de la gélose à une concentration de 0,4 à 1,1 pour 100 en poids de la perle.

2. Perle selon la revendication 1, ayant une masse de 0,1 à 2,5 grammes.

3. Perle selon la revendication 2, ayant une masse de 0,4 à 1 gramme.

4. Trousse ou plaquette à utiliser pour des soins dentaires, comprenant au moins une perle de gel semi-solide selon la revendication 1, logée dans un compartiment à l'intérieur de la plaquette.

5. Trousse ou plaquette selon la revendication 4, comprenant en outre un outil d'application comportant des moyens destinés à venir au contact d'une pluralité de surfaces des dents de la personne afin que, après rupture d'une perle et libération du/des principes actifs au voisinage immédiat des dents, l'emploi de l'outil contribue au nettoyage des dents.

6. Trousse ou plaquette selon la revendication 4, dans lequel chaque compartiment comporte un creux formé dans une feuille déformable et un moyen couvrant pour couvrir le creux.

7. Procédé de fabrication de perles selon la revendication 1, lequel procédé comprenant les étapes consistant à mélanger de l'eau avec au moins un des principes actifs phamaceutiquement compatibles, ainsi qu'avec au moins un gélifiant, à une température suffisante pour faire fondre le gélifiant, puis à refroidir le mélange jusqu'à une température inférieure à laquelle la composition est encore en fusion, puis éventuellement à ajouter et incorporer par mélange au moins un autre principe actif, puis à expulser le mélange depuis un orifice.

8. Procédé selon la revendication 7, dans lequel, après expulsion depuis l'orifice, le mélange est refroidi afin de provoquer une solidification, puis le mélange solidifié est divisé en une ou plusieurs perles homogènes ayant chacune une masse voulue.

9. Procédé selon la revendication 7, dans lequel le dispositif employé lors de la fabrication comprend un moyen pour déposer une quantité dosée de la composition fondue, à partir de l'orifice, dans au moins un d'une pluralité de creux formés dans une feuille déformable, un moyen pour permettre à la composition fondue de se solidifier, en formant de la sorte au moins une perle coulée en place, et un moyen pour enfermer la/les perles en plaçant une feuille couvrante sur le creux, si bien qu'une plaquette contenant au moins une perle coulée en place est ainsi réalisée.

10. Procédé selon la revendication 7, dans lequel le dispositif employé pour la fabrication comprend un moyen à inclure dans la plaquette ou la trousse de brosses à dents.
